# EUROPEAN PATENT SPECIFICATION

(11) **EP 3 119 383 B1**
(45) Date of publication and mention of the grant of the patent: **29.07.2020**
(21) Application number: 15780933.6
(22) Date of filing: 05.06.2015
(51) Int. Cl.: A61K 36/31, A61K 31/07, A61K 31/575, A61K 31/59, A61K 36/53, A61K 36/55, A61P 9/00

(54) **COMPOSITION FOR TREATMENT OF OXIDATIVE STRESS AND CARDIOVASCULAR DISEASE**
ZUSAMMENSETZUNG ZUR BEHANDLUNG VON OXIDATIVEM STRESS UND KARDIOVASKULÄREN ERKRANKUNG
COMPOSITION POUR LE TRAITEMENT DU STRESS OXYDATIF ET DE MALADIES CARDIO-VASCULAIRES

(30) Priority: 22.05.2015 US 201561165669 P
(43) Date of publication of application: 25.01.2017
(73) Proprietor: Team Foods Colombia S.A., Bogotá (CO)
(72) Inventor: BETANCOURT VILLAMIZAR, Eddy, Carolina, Bogotá D.C (CO); MURCILLO ROJAS, Juan, Fernando, Bogotá D.C. (CO); BETENCOURT CORTÉS, Rubén, Dario, Bogotá D.C. (CO)
(74) Representative: Dehns
(86) International application number: PCT/IB2015/000870
(87) International publication number: WO 2016/189345

(56) References cited:
- US-A1- 2008 305 096
- DATABASE WPI Week 201369 Thomson Scientific, London, GB; AN 2013-S28219 XP002751115, & CN 103 156 003 A (FENGYI SHANGHAI BIOTECHNOLOGY RES & DEV) 19 June 2013 (2013-06-19)
- DATABASE WPI Week 201057 Thomson Scientific, London, GB; AN 2010-K02601 XP002751116, & CN 101 766 236 A (SOUTHSEAS SPECIALTY FATS IND SHANGHAI CO) 7 July 2010 (2010-07-07)
- DATABASE WPI Week 201055 Thomson Scientific, London, GB; AN 2010-K02602 XP002751117, & CN 101 766 235 A (SOUTHSEAS SPECIALTY FATS IND SHANGHAI CO) 7 July 2010 (2010-07-07)
- DATABASE WPI Week 201061 Thomson Scientific, London, GB; AN 2010-K00032 XP002751118, & CN 101 755 926 A (ZHONGLIANG BEIHAI OIL & CEREAL IND TIANJ) 30 June 2010 (2010-06-30)
- LEAH G. GILLINGHAM ET AL: "High-oleic rapeseed (canola) and flaxseed oils modulate serum lipids and inflammatory biomarkers in hypercholesterolaemic subjects", BRITISH JOURNAL OF NUTRITION, vol. 105, no. 03, 29 September 2010 (2010-09-29), pages 417-427, XP055229367, UK ISSN: 0007-1145, DOI: 10.1017/S0007114510003697

## Description

### BACKGROUND OF INVENTION

Oxidative stress and cardiovascular diseases are known.

Oil compositions are also known in the art with cerebroprotective and cardioprotective effects, see for example CN 103 156 003 A, US 2008/305096 A1, CN 101 766 235 A, CN 101 766 236 A and CN 101 755 926 A. A study of novel dietary oils is also reported in Gillingham et al., British Journal of Nutrition (2011), 105, pp. 417-427.

### TECHNICAL FIELD

The present invention relates to compositions for treatment of inflammation related to oxidative stress and cardiovascular disease.

### BRIEF SUMMARY OF INVENTION

In some embodiments, the present invention is a composition consisting of 35 to 50 weight percent of a first vegetable oil having a saturated fatty acid content of 4% to 12%; 35 to 50 weight percent of a second vegetable oil having an alpha-linolenic acid content of 45% to 65%; 0.005 to 0.15 weight percent of at least one of Vitamin A and Vitamin D; 2 to 10 weight percent of at least one phytosterol; and 0.02 to 0.2 weight percent of antioxidant, wherein the antioxidant is selected from the group consisting of tocopherol, ascorbyl palmitate, Rosemary extract and mixtures thereof.

In some embodiments, the first vegetable oil is selected from the group consisting of canola oil, soybean oil, corn oil and sunflower oil. In some embodiments, the second vegetable oil is selected from the group consisting of flax seed oil, chia oil, and sacha inchi oil.

In some embodiments, the composition comprises 0.005 to 0.15 weight percent of Vitamin A and Vitamin D. In some embodiments, the at least one phytosterol comprises at least one vegetable sterol. In some embodiments, the composition comprises 40 to 50 weight percent of the first vegetable oil.

In some embodiments, the composition comprises 40 to 50 weight percent of the second vegetable oil. In some embodiments, the composition comprises 5 to 10 weight percent of the at least one phytosterol. In some embodiments, the composition comprises 0.1 to 0.2 weight percent of the antioxidant.

In some embodiments, the effective amount of the composition of the invention comprises 8 to 11 grams per dose. In some embodiments, the effective amount comprises 2 doses per day.

In some embodiments, the present invention is a composition according to any of the embodiments detailed herein for use in treatment of inflammation related to oxidative stress. In some embodiments, the present invention is a composition according to any of the embodiments detailed herein for use in treatment of cardiovascular disease.

In some embodiments, a composition according to any of the embodiments detailed herein is used so as to result in reduction of the levels of C reactive protein (PCR) under 1.0 mg/ L blood.

### BRIEF DESCRIPTION OF THE DRAWINGS

FIG. 1 illustrates features of some embodiments of the present invention.
FIG. 2 illustrates features of some embodiments of the present invention.

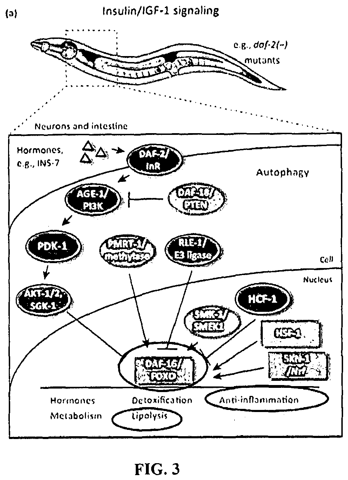

FIG. 3 illustrates features of some embodiments of the present invention.
FIG. 4 illustrates features of some embodiments of the present invention.
FIG. 5 illustrates features of some embodiments of the present invention.
FIG. 6 illustrates features of some embodiments of the present invention.
FIG. 7 illustrates features of some embodiments of the present invention.

The figures constitute a part of this specification and include illustrative embodiments of the present invention and illustrate various objects and features thereof. Further, the figures are not necessarily to scale, some features may be exaggerated to show details of particular components. In addition, any measurements, specifications and the like shown in the figures are intended to be illustrative, and not restrictive. Therefore, specific structural and functional details disclosed herein are not to be interpreted as limiting, but merely as a representative basis for teaching one skilled in the art to variously employ the present invention.

### DETAILED DESCRIPTION

The present invention will be further explained with reference to the attached drawings, wherein like structures are referred to by like numerals throughout the several views. The drawings shown are not necessarily to scale, with emphasis instead generally being placed upon illustrating the principles of the present invention. Further, some features may be exaggerated to show details of particular components.

The figures constitute a part of this specification and include illustrative embodiments of the present invention and illustrate various objects and features thereof. Further, the figures are not necessarily to scale, some features may be exaggerated to show details of particular components. In addition, any measurements, specifications and the like shown in the figures are intended to be illustrative, and not restrictive. Therefore, specific structural and functional details disclosed herein are not to be interpreted as limiting, but merely as a representative basis for teaching one skilled in the art to variously employ the present invention.

Among those benefits and improvements that have been disclosed, other objects and advantages of this invention will become apparent from the following description taken in conjunction with the accompanying figures. Detailed embodiments of the present invention are disclosed herein; however, it is to be understood that the disclosed embodiments are merely illustrative of the invention that may be embodied in various forms. In addition, each of the examples given in connection with the various embodiments of the invention which are intended to be illustrative, and not restrictive.

Throughout the specification and claims, the following terms take the meanings explicitly associated herein, unless the context clearly dictates otherwise. The phrases "in one embodiment" and "in some embodiments" as used herein do not necessarily refer to the same embodiment(s), though it may. Furthermore, the phrases "in another embodiment" and "in some other embodiments" as used herein do not necessarily refer to a different embodiment, although it may. Thus, as described below, various embodiments of the invention may be readily combined, without departing from the scope of the invention.

In addition, as used herein, the term "or" is an inclusive "or" operator, and is equivalent to the term "and/or," unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on".

In some embodiments, the present invention is a composition consisting of 35 to 50 weight percent of a first vegetable oil having a saturated fatty acid content of 4% to 12%; 35 to 50 weight percent of a second vegetable oil having an alpha-linolenic acid content of 45 % to 65%; 0.005 to 0.15 weight percent of at least one of Vitamin A and Vitamin D; 2 to 10 weight percent of at least one phytosterol; and 0.02 to 0.2 weight percent of antioxidant, wherein the antioxidant is selected from the group consisting of tocopherol, ascorbyl palmitate, Rosemary extract and mixtures thereof.

In some embodiments, the first vegetable oil is selected from the group consisting of canola oil, soybean oil, com oil and sunflower oil. In some embodiments, the second vegetable oil is selected from the group consisting of flax seed oil, chia oil, and sacha inchi oil.

In some embodiments, the composition comprises 0.005 to 0.15 weight percent of Vitamin A and Vitamin D. In some embodiments, the at least one phytosterol comprises at least one vegetable sterol. In some embodiments, the composition comprises 40 to 50 weight percent of the first vegetable oil.

In some embodiments, the composition comprises 40 to 50 weight percent of the second vegetable oil. In some embodiments, the composition comprises 5 to 10 weight percent of the at least one phytosterol. In some embodiments, the composition comprises 0.1 to 0.2 weight percent of the antioxidant.

In some embodiments, the effective amount of the composition of the invention comprises 8 to 11 grams per dose. In some embodiments, the effective amount comprises 2 doses per day.

In some embodiments, the present invention is a composition according to any of the embodiments detailed herein for use in treatment of inflammation related to oxidative stress. In some embodiments, the present invention is a composition according to any of the embodiments detailed herein for use in treatment of cardiovascular disease.

In some embodiments, a composition according to any of the embodiments detailed herein is used so as to result in reduction of the levels of C reactive protein (PCR) under 1.0 mg/ L blood.

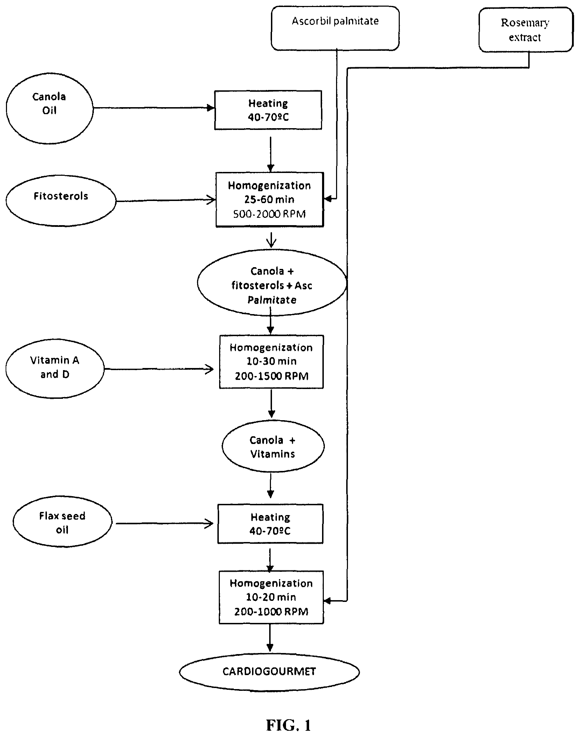

A method of making the composition according to an embodiment of the present invention is shown in Figure 1.

In some embodiments, the composition comprises 35 to 45 weight percent of a first vegetable oil having a saturated fatty acid content of 4% to 12%. In some embodiments, the composition comprises 35 to 40 weight percent of a first vegetable oil having a saturated fatty acid content of 4% to 12%. In some embodiments, the composition comprises 40 to 50 weight percent of a first vegetable oil having a saturated fatty acid content of 4% to 12%. In some embodiments, the composition comprises 45 to 50 weight percent of a first vegetable oil having a saturated fatty acid content of 4% to 12%. In some embodiments, the composition comprises 40 to 45 weight percent of a first vegetable oil having a saturated fatty acid content of 4% to 12%.

In some embodiments, the composition comprises 35 to 50 weight percent of a first vegetable oil having a saturated fatty acid content of 4% to 10%. In some embodiments, the composition comprises 35 to 50 weight percent of a first vegetable oil having a saturated fatty acid content of 4% to 8%. In some embodiments, the composition comprises 35 to 50 weight percent of a first vegetable oil having a saturated fatty acid content of 4% to 6%. In some embodiments, the composition comprises 35 to 50 weight percent of a first vegetable oil having a saturated fatty acid content of 6% to 12%. In some embodiments, the composition comprises 35 to 50 weight percent of a first vegetable oil having a saturated fatty acid content of 8% to 12%. In some embodiments, the composition comprises 35 to 50 weight percent of a first vegetable oil having a saturated fatty acid content of 10% to 12%. In some embodiments, the composition comprises 35 to 50 weight percent of a first vegetable oil having a saturated fatty acid content of 6% to 10%.

In some embodiments, the composition further comprises 35 to 45 weight percent of a second vegetable oil having an alpha-linolenic acid content of 45% to 65% In some embodiments, the composition further comprises 35 to 40 weight percent of a second vegetable oil having an alpha-linolenic acid content of 45% to 65%.

In some embodiments, the composition further comprises 40 to 50 weight percent of a second vegetable oil having an alpha-linolenic acid content of 45% to 65%. In some embodiments, the composition further comprises 45 to 50 weight percent of a second vegetable oil having an alpha-linolenic acid content of 45% to 65%. In some embodiments, the composition further comprises 50 to 60 weight percent of a second vegetable oil having an alpha-linolenic acid content of 45% to 65%.

In some embodiments, the composition further comprises 35 to 50 weight percent of a second vegetable oil having an alpha-linolenic acid content of 45% to 60%. In some embodiments, the composition further comprises 35 to 50 weight percent of a second vegetable oil having an alpha-linolenic acid content of 45% to 55%. In some embodiments, the composition further comprises 35 to 50 weight percent of a second vegetable oil having an alpha-linolenic acid content of 45% to 50%.

In some embodiments, the composition further comprises 35 to 50 weight percent of a second vegetable oil having an alpha-linolenic acid content of 50% to 65%. In some embodiments, the composition further comprises 35 to 50 weight percent of a second vegetable oil having an alpha-linolenic acid content of 55% to 65%. In some embodiments, the composition further comprises 35 to 50 weight percent of a second vegetable oil having an alpha-linolenic acid content of 60% to 65%. In some embodiments, the composition further comprises 35 to 50 weight percent of a second vegetable oil having an alpha-linolenic acid content of 50% to 60%.

In some embodiments, the composition further comprises 0.005 to 0.1 weight percent of at least one of Vitamin A and Vitamin D. In some embodiments, the composition further comprises 0.005 to 0.05 weight percent of at least one of Vitamin A and Vitamin D. In some embodiments, the composition further comprises 0.005 to 0.01 weight percent of at least one of Vitamin A and Vitamin D.

In some embodiments, the composition further comprises 0.01 to 0.15 weight percent of at least one of Vitamin A and Vitamin D. In some embodiments, the composition further comprises 0.05 to 0.15 weight percent of at least one of Vitamin A and Vitamin D. In some embodiments, the composition further comprises 0.1 to 0.15 weight percent of at least one of Vitamin A and Vitamin D. In some embodiments, the composition further comprises 0.01 to 0.1 weight percent of at least one of Vitamin A and Vitamin D.

In some embodiments, the composition further comprises 2 to 8 weight percent of at least one phytosterol. In some embodiments, the composition further comprises 2 to 6 weight percent of at least one phytosterol. In some embodiments, the composition further comprises 2 to 4 weight percent of at least one phytosterol.

In some embodiments, the composition further comprises 4 to 10 weight percent of at least one phytosterol. In some embodiments, the composition further comprises 6 to 10 weight percent of at least one phytosterol. In some embodiments, the composition further comprises 8 to 10 weight percent of at least one phytosterol. In some embodiments, the composition further comprises 4 to 8 weight percent of at least one phytosterol. In some embodiments, the composition further comprises 4 to 5 weight percent of at least one phytosterol.

In some embodiments, the composition further comprises 0.02 to 0.15 weight percent of antioxidant. In some embodiments, the composition further comprises 0.02 to 0.1 weight percent of antioxidant. In some embodiments, the composition further comprises 0.02 to 0.05 weight percent of antioxidant. In some embodiments, the composition further comprises 0.05 to 0.2 weight percent of antioxidant. In some embodiments, the composition further comprises 0.1 to 0.2 weight percent of antioxidant. In some embodiments, the composition further comprises 0.15 to 0.2 weight percent of antioxidant. In some embodiments, the composition further comprises 0.05 to 0.15 weight percent of antioxidant.

Oxidative stress is a disturbance in the prooxidant-antioxidant balance in favor of the former, leading to potential damage. Other indicators of oxidative stress may include damaged DNA bases, protein oxidation products, and lipid peroxidation products. Some conditions related to oxidative stress and cardiovascular risk are free radical accumulation (measured by a nuclear stress test), vascular inflammation (measured by echocardiogram) and general inflammation (measured by the level of C reactive protein (PCR) in the blood).

In embodiments, the effective amount for treatment of inflammation related to oxidative stress comprises 8 to 10 grams per dose. In embodiments, the effective amount comprises 8 to 9 grams per dose. In embodiments, the effective amount comprises 9 to 11 grams per dose. In embodiments, the effective amount comprises 10 to 11 grams per dose. In embodiments, the effective amount comprises 9 to 10 grams per dose.

In embodiments, the effective amount comprises 16 to 20 grams per day. In embodiments, the effective amount comprises 16 to 18 grams per day. In embodiments, the effective amount comprises 18 to 22 grams per day. In embodiments, the effective amount comprises 20 to 22 grams per day. In embodiments, the effective amount comprises 18 to 20 grams per day.

In embodiments, the effective amount comprises 1 dose per day. In embodiments, the effective amount comprises 2 doses per day. In embodiments, the effective amount comprises 3 doses per day. In embodiments, the effective amount comprises 4 doses per day. In embodiments, the effective amount comprises 5 doses per day. In embodiments, the effective amount comprises 6 doses per day. In embodiments, the effective amount comprises 7 doses per day. In embodiments, the effective amount comprises more than 7 doses per day.

Cardiovascular disease is the broad term for problems with the heart and blood vessels. Conditions related to cardiovascular diseases may include coronary heart disease (measured by an electrocardiogram (ECG)), heart failure (measured by an echocardiogram), arrhythmias (measured by a Holter monitor), peripheral artery disease (measured by arteriography), and/or stroke (measure by magnetic resonance angiography (MRA)).

In embodiments, the effective amount for treatment of cardiovascular disease comprises 8 to 10 grams per dose. In embodiments, the effective amount comprises 8 to 9 grams per dose. In embodiments, the effective amount comprises 9 to 11 grams per dose. In embodiments, the effective amount comprises 10 to 11 grams per dose. In embodiments, the effective amount comprises 9 to 10 grams per dose.

In embodiments, the effective amount comprises 16 to 20 grams per day. In embodiments, the effective amount comprises 16 to 18 grams per day. In embodiments, the effective amount comprises 18 to 22 grams per day. In embodiments, the effective amount comprises 20 to 22 grams per day. In embodiments, the effective amount comprises 18 to 20 grams per day.

In embodiments, the effective amount comprises 1 dose per day. In embodiments, the effective amount comprises 2 doses per day. In embodiments, the effective amount comprises 3 doses per day. In embodiments, the effective amount comprises 4 doses per day. In embodiments, the effective amount comprises 5 doses per day. In embodiments, the effective amount comprises 6 doses per day. In embodiments, the effective amount comprises 7 doses per day. In embodiments, the effective amount comprises more than 7 doses per day.

### Non-limiting Examples

The following examples are intended to illustrate the invention and should not be construed as limiting the invention in any way.

The non-limiting examples include testing of the anti-inflammatory properties of the lipid extract CARDIOGOURMET of an embodiment of the present invention in a *C*. *elegans* model. The examples evaluate the antioxidant activity, oxidative stress and inflammatory signaling related to the lipid extract CARDIOGOURMET of an embodiment of the present invention. Moreover, the examples analyze if the molecular target of the lipid extract CARDIOGOURMET of an embodiment of the present invention is the insulin signaling pathway (IGF-I) and the transcriptional factor DAF-16, as previously described for anti-inflammatory systems.

### Example 1 -- Antioxidant effect

The experiments in this example were carried out by culturing synchronized nematodes age of the wild strain in different conditions N2 supply:
NG (standard culture media);
NG + Vitamin C (positive control);
100% Ascorbic Acid;
NG + lipid blend CONTROLG (negative control);
70-80% Canola oil, 20-30% Sunflower oil, 100-200 µg Vitamin A, 0.5-2µg Vitamin D and 1-3 mg Vitamin E;
NG + lipid blend CARDIOGOURMET (compound tested); and
30-50% Canola oil, 60-70% flaxed oil, 5-10% phytosterols, 100-200 µg Vitamin A and 0.5-2µg Vitamin D.

The compositions of CARDIOGOURMET and CONTROL G are detailed in Tables 1 and 2.

**TABLE 1**

| **CARDIOGOURMET** | |
|---|---|
| Raw Material | % |
| Canola oil | 46.59 |
| Flax seed oil | 45.30 |
| Phytosterols | 8.00 |
| Rosemary Extract | 0.10 |
| Vitamin A and D | 0.01 |

**TABLE 2**

| **CONTROL G** | |
|---|---|
| Raw Material | % |
| Canola Oil | 60.00 |
| Sunflower oil | 39.95 |
| Alpha-Tocopherol | 0.04 |
| Vitamin A and D | 0.007 |
| TBHQ (antioxidant) | 0.0012 |

In this example, worms were incubated at 20°C and after 5 days were subjected to an oxidative stress with hydrogen peroxide at 2mM for 5 hours. A survival count was then performed on each condition.

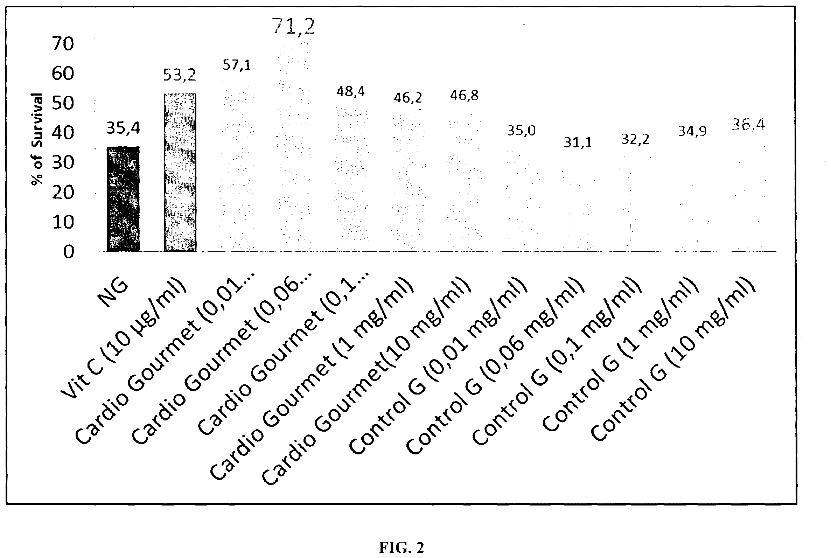

Five different concentrations of each sample (CONTROL G and CARDIOGOURMET) were tested at 0.01 mg/mL, 0.06 mg/mL. 0.1 mg/mL, 1 mg/mL and 10 mg/mL. The antioxidant activity in *C*. *elegans.* survival rate after oxidative stress with hydrogen peroxide at 2mM of each sample is shown in Figure 2.

As noted in Figure 2, an antioxidant effect with CARDIOGOURMET was observed as illustrated by increased nematode survival after acute oxidative stress. In contrast, no antioxidant effect was observed in the case of the CONTROL G.

### Example 2 -- Molecular Pathway

An additional experiment was conducted with *C. elegans* strain DAF-16 IIS-regulated mutant. Life expectancy tests were performed on two conditions: NG (control) and CARDIOGOURMET. As noted in Figure 2, the highest survival rate occurred in example 1 with a dose of 0.06 mg/ml of CARDIOGOURMET. Accordingly, a dose of 0.06 mg/ml of CARDIOGOURMET was also used for this example.

Survival curves where examined to assess if the sample produces an increase in the longevity of DAF-16 mutants. The results of assessment then suggest whether the condition acts to modulate the activity of the IIS path and thus, has anti-inflammatory activity and thus, would potentially be effective in the treatment of oxidative stress and/or cardiovascular disease.

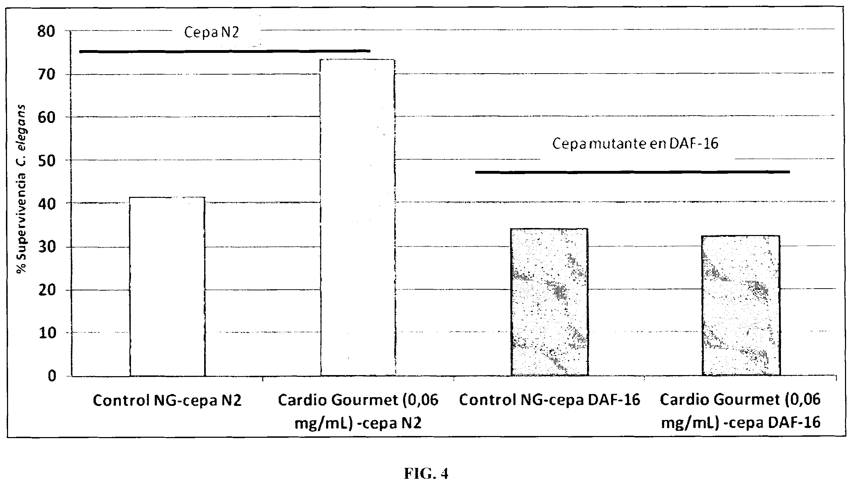

Acute oxidative stress tests performed with N2 and DAF-16 mutant strains used hydrogen peroxide at 1.75 mM. The effect of total loss of antioxidant protective phenotype in the mutant strain in DAF-16 in the presence of CARDIOGOURMET is shown in Figure 4.

As shown in Figure 4, CARDIOGOURMET exhibited a complete loss of antioxidant protective phenotype mutant strain in DAF-16. The results suggest CARDIOGOURMET exhibits antioxidant activity and may require transcriptional factor for such activity. The results further illustrate the anti-inflammatory potential of CARDIOGOURMET and thus, the potential effectiveness for treatment of oxidative stress and/or cardiovascular disease.

### Example 3 - Reduction of Corporal Fat

Assays for measuring body fat in *C*. *elegans* were done by staining with Nile Red (λ ex 480nm . λ Em 571nm). This example was carried out by culturing synchronized nematodes age of the N2 strain NG (negative control) medium supplemented with CARDIOGOURMET (at the five concentrations detailed in Example 1). Fat quantification was performed in young adults by measuring fluorescence spectrofluorimeter. The relative percentage of fluorescence was determined in the CARDIOGOURMET versus control nematodes:
NG - DMSO
NG + Orlistat (positive control)
100% tetrahidrolipstatina
NG + lipid blend CARDIOGOURMET (compound tested)
30-50% Canola oil, 60-70% flaxseed oil, 5-10% phytosterols, 100-200 µg Vitamin A and 0.5-2µg Vitamin D

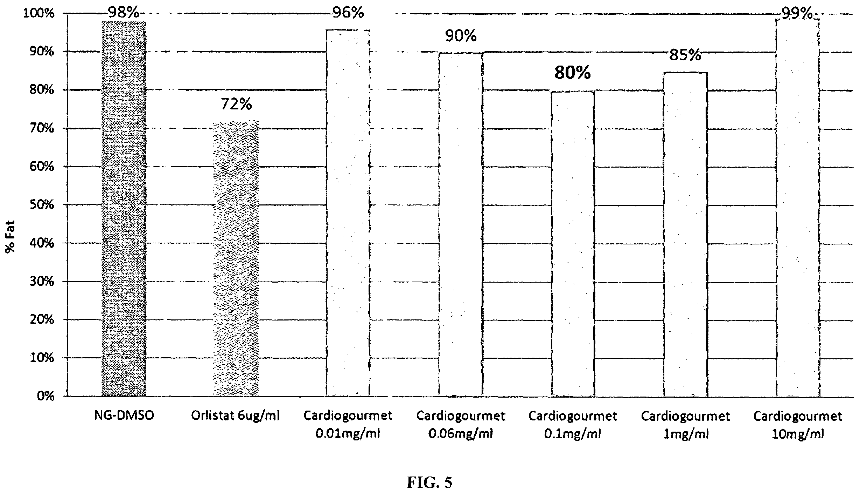

The dose of 0.1 mg/mL (Figure 5) causes a reduction of 20% of the fat in the nematode (relative to normal conditions NG - DMSO which result in only a 2% reduction in the nematode fat) and thus, shows potential effectiveness for treatment of oxidative stress and/or cardiovascular disease.

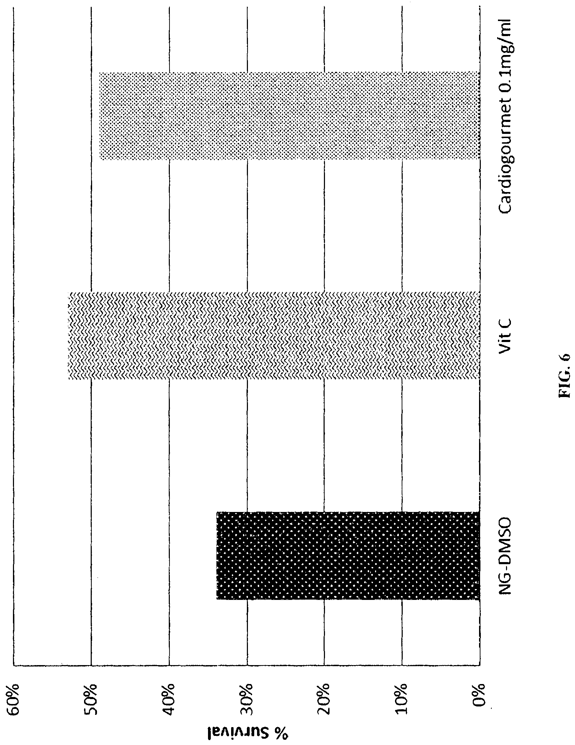

This dose also produced an antioxidant effect as illustrated by an increase of 13% survival in *C*. *elegans* as shown in Figure 6 and thus, shows potential effectiveness for treatment of oxidative stress and/or cardiovascular disease.

### Additional Details Regarding Application of C. elegans Model

*C. elegans* is a free-living nematode, approximately 1 mm in length, which exists as either a self-fertilizing hermaphrodite or as a male. *C*. *elegans* genome has been fully sequenced which has revealed that about 80% of *C*. *elegans* genes have human homologs and at least 42% of human disease-related genes have a C. elegans homolog.

The nematode *Caenorhabditis elegans* has become established as a major experimental organism with applications to many biomedical research areas. The body wall muscle cells are a useful model for the study of human cardiomyocytes and their homologous structures and proteins. Because of the small size of this nematode (1-mm-long adults), a heart and circulatory system are not required. The close homology of proteins and structures of interest justify the study of nematode body wall muscle as a way to understand human heart muscle.

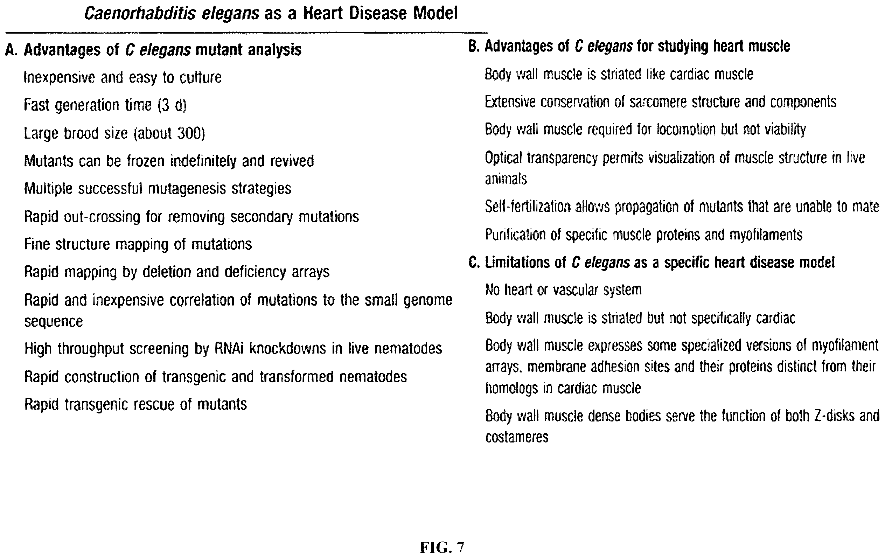

In addition, *C*. *elegans* has become an excellent model for screening of compounds for therapeutic purposes. Regarding inflammation, *C*. *elegans* has recently been used to study an anti-inflammatory non-steroidal: Celecoxib ®R. The results showed that the drug requires the activity of DAF-16, FOXO transcription factor that regulates longevity in response to the signaling pathway insulin /IGF- I (IIS). All this suggests that IIS is a key target for the search for compounds with potential anti -inflammatory properties. Figure 7 illustrates some additional features of the *C. elegans* model.

### Example 3

The ability of the one embodiment of the composition of the present invention to treat oxidative stress and/or cardiovascular disease in human subjects is evaluated in this example. Human subjects are treated with a composition detailed in Table 3:

**TABLE 3**

| **CARDIOGOURMET** | |
|---|---|
| Raw Material | % |
| Canola oil | 46.59 |
| Flax seed oil | 45.30 |
| Phytosterols | 8.00 |
| Rosemary Extract | 0.10 |
| Vitamin A and D | 0.01 |

The treatment regimen is 9 grams per dose and 2 doses per day. The levels of C reactive protein (PCR) in the human subject's blood are measured before and after treatment. Treated humans show a reduction in the levels of C reactive protein (PCR) to under 1.0 mg/L in the treated human subject's blood compared to a non-treated human or the human subject before treatment. Thus, the example shows treatment of cardiovascular disease and oxidative stress in the treated human subject compared to a non-treated human or the human subject before treatment.

While a number of embodiments of the present invention have been described, it is understood that these embodiments are illustrative only, and not restrictive, and that many modifications may become apparent to those of ordinary skill in the art. Further still, the various steps may be carried out in any desired order (and any desired steps may be added and/or any desired steps may be eliminated).

## Claims

1. A composition consisting of:
(i) 35 to 50 weight percent of a first vegetable oil having a saturated fatty acid content of 4% to 12%;
(ii) 35 to 50 weight percent of a second vegetable oil having an alpha-linolenic acid content of 45 % to 65%;
(iii) 0.005 to 0.15 weight percent of at least one of Vitamin A and Vitamin D;
(iv) 2 to 10 weight percent of at least one phytosterol; and
(v) 0.02 to 0.2 weight percent of antioxidant,
wherein the antioxidant is selected from the group consisting of tocopherol, ascorbyl palmitate, Rosemary extract and mixtures thereof.

2. The composition of claim 1, wherein the first vegetable oil is selected from the group consisting of canola oil, soybean oil, corn oil and sunflower oil.

3. The composition according to claim 1 or claim 2, wherein the second vegetable oil is selected from the group consisting of flax seed oil, chia oil, and sacha inchi oil.

4. The composition of claim 1, comprising 0.005 to 0.15 weight percent of Vitamin A and Vitamin D.

5. The composition of claim 1, wherein the at least one phytosterol comprises at least one vegetable sterol.

6. The composition of claim 1, wherein the composition comprises 40 to 50 weight percent of the first vegetable oil.

7. The composition of claim 1, wherein the composition comprises 40 to 50 weight percent of the second vegetable oil.

8. The composition of claim 1, wherein the composition comprises 5 to 10 weight percent of the at least one phytosterol.

9. The composition according to any one of claims 1 to 8, wherein the composition comprises 0.1 to 0.2 weight percent of the antioxidant.

10. A composition according to any one of claims 1 to 9 for use in treatment of inflammation related to oxidative stress.

11. A composition according to any one of claims 1 to 9 for use in treatment of cardiovascular disease.

## Patentansprüche

1. Zusammensetzung, bestehend aus:
(i) 35 bis 50 Gewichtsprozent eines ersten Pflanzenöls, das einen Gehalt an gesättigter Fettsäure von 4 % bis 12 % aufweist;
(ii) 35 bis 50 Gewichtsprozent eines zweiten Pflanzenöls, das einen Gehalt an alpha-Linolensäure von 45 % bis 65 % aufweist;
(iii) 0,005 bis 0,15 Gewichtsprozent von mindestens einem von Vitamin A und Vitamin D;
(iv) 2 bis 10 Gewichtsprozent von mindestens einem Phytosterol; und
(v) 0,02 bis 0,2 Gewichtsprozent eines Antioxidationsmittels,
wobei das Antioxidationsmittel ausgewählt ist aus der Gruppe, bestehend aus Tocopherol, Ascorbylpalmitat, Rosmarinextrakt und Gemischen daraus.

2. Zusammensetzung nach Anspruch 1, wobei das erste Pflanzenöl ausgewählt ist aus der Gruppe, bestehend aus Canolaöl, Sojabohnenöl, Maisöl und Sonnenblumenöl.

3. Zusammensetzung nach Anspruch 1 oder Anspruch 2, wobei das zweite Pflanzenöl ausgewählt ist aus der Gruppe, bestehend aus Leinsamenöl, Chiaöl und Sacha Inchi-Öl.

4. Zusammensetzung nach Anspruch 1, umfassend 0,005 bis 0,15 Gewichtsprozent an Vitamin A und Vitamin D.

5. Zusammensetzung nach Anspruch 1, wobei das mindestens eine Phytosterol mindestens ein Pflanzensterol umfasst.

6. Zusammensetzung nach Anspruch 1, wobei die Zusammensetzung 40 bis 50 Gewichtsprozent des ersten Pflanzenöls umfasst.

7. Zusammensetzung nach Anspruch 1, wobei die Zusammensetzung 40 bis 50 Gewichtsprozent des zweiten Pflanzenöls umfasst.

8. Zusammensetzung nach Anspruch 1, wobei die Zusammensetzung 5 bis 10 Gewichtsprozent des mindestens einen Phytosterols umfasst.

9. Zusammensetzung nach einem der Ansprüche 1 bis 8, wobei die Zusammensetzung 0,1 bis 0,2 Gewichtsprozent des Antioxidationsmittels umfasst.

10. Zusammensetzung nach einem der Ansprüche 1 bis 9 zur Verwendung bei der Behandlung von Entzündung im Zusammenhang mit oxidativem Stress.

11. Zusammensetzung nach einem der Ansprüche 1 bis 9 zur Verwendung bei der Behandlung von kardiovaskulärer Erkrankung.

## Revendications

1. Composition constituée par :
(i) 35 à 50 pour cent en poids d'une première huile végétale présentant une teneur en acides gras saturés de 4 % à 12 % ;
(ii) 35 à 50 pour cent en poids d'une seconde huile végétale présentant une teneur en acide alphalinolénique de 45 % à 65 % ;
(iii) 0,005 à 0,15 pour cent en poids d'au moins l'une parmi la vitamine A et la vitamine D ;
(iv) 2 à 10 pour cent en poids d'au moins un phytostérol ; et
(v) 0,02 à 0,2 pour cent en poids d'antioxydant,
dans laquelle l'antioxydant est sélectionné dans le groupe constitué par le tocophérol, le palmitate d'ascorbyle, un extrait de romarin et des mélanges de ceux-ci.

2. Composition selon la revendication 1, dans laquelle la première huile végétale est sélectionnée dans le groupe constitué par l'huile de colza, l'huile de soja, l'huile de maïs et l'huile de tournesol.

3. Composition selon la revendication 1 ou la revendication 2, dans laquelle la seconde huile végétale est sélectionnée dans le groupe constitué par l'huile de lin, l'huile de chia et l'huile de sacha inchi.

4. Composition selon la revendication 1, comprenant 0,005 à 0,15 pour cent en poids de vitamine A et de vitamine D.

5. Composition selon la revendication 1, dans laquelle l'au moins un phytostérol comprend au moins un stérol végétal.

6. Composition selon la revendication 1, dans laquelle la composition comprend 40 à 50 pour cent en poids de la première huile végétale.

7. Composition selon la revendication 1, dans laquelle la composition comprend 40 à 50 pour cent en poids de la seconde huile végétale.

8. Composition selon la revendication 1, dans laquelle la composition comprend 5 à 10 pour cent en poids de l'au moins un phytostérol.

9. Composition selon l'une quelconque des revendications 1 à 8, dans laquelle la composition comprend 0,1 à 0,2 pour cent en poids de l'antioxydant.

10. Composition selon l'une quelconque des revendications 1 à 9 pour une utilisation dans le traitement d'une inflammation liée au stress oxydatif.

11. Composition selon l'une quelconque des revendications 1 à 9 pour une utilisation dans le traitement d'une maladie cardiovasculaire.
